## Europäisches Patentamt
## European Patent Office
## Office européen des brevets

(11) Publication number: **0 107 876**
**B1**

# EUROPEAN PATENT SPECIFICATION

(45) Date of publication of patent specification: **21.03.90**

(51) Int. Cl.⁵: **C 07 C 2/76, B 01 J 29/04**

(21) Application number: **83201421.1**

(22) Date of filing: **04.10.83**

(54) Process for the preparation of an aromatic hydrocarbon mixture.

(30) Priority: **28.10.82 NL 8204167**
**30.11.82 NL 8204632**

(43) Date of publication of application:
**09.05.84 Bulletin 84/19**

(45) Publication of the grant of the patent:
**21.03.90 Bulletin 90/12**

(84) Designated Contracting States:
**BE DE FR GB IT NL**

(56) References cited:
**EP-A-0 021 475**
**EP-A-0 024 147**
**EP-A-0 089 795**
**DE-A-2 755 770**
**FR-A-2 374 283**

(73) Proprietor: **SHELL INTERNATIONALE**
**RESEARCH MAATSCHAPPIJ B.V.**
**Carel van Bylandtlaan 30**
**NL-2596 HR Den Haag (NL)**

(72) Inventor: **Kieffer, Eduard Philip**
**Badhuisweg 3**
**NL-1031 CM Amsterdam (NL)**

(74) Representative: **Aalbers, Onno et al**
**P.O. Box 302**
**NL-2501 CH The Hague (NL)**

Courier Press, Leamington Spa, England.

**Description**

The present invention relates to a process for the preparation of an aromatic hydrocarbon mixture, in which paraffins with two, three or four carbon atoms per molecule or aliphatic hydrocarbon mixtures consisting more than 50%w of said paraffins are contacted with a catalyst containing a crystalline gallium silicate which

a) has been prepared by crystallization starting from an aqueous mixture which contains one or more compounds of an alkali metal, one or more organic nitrogen compounds which contain an organic cation or from which an organic cation is formed during the preparation of the silicate, one or more silicon compounds, one or more gallium compounds and, if desired, one or more compounds of a trivalent metal Y chosen from the group formed by aluminium, iron, cobalt and chromium in such quantities that in the formula which represents the composition of the silicate expressed in moles of the oxides, the $SiO_2/Ga_2O_3$ molar ratio is 25—250 and the $Y_2O_3/Ga_2O_3$ molar ratio is lower than 1, and

b) after one hour's calcination in air at 500°C has an X-ray powder diffraction pattern in which the strongest lines are the four lines mentioned in Table A,

TABLE A
d(Å)

| |
| --- |
| 11.1 ±0.2 |
| 10.0 ±0.2 |
| 3.84±0.07 |
| 3.72±0.06 |

which catalyst has been subjected at least once to a two-step treatment involving a step in which the catalyst is contacted at a temperature of 350—700°C with a hydrocarbon or a mixture of hydrocarbons until in less than 5 hours 0.1—5%w of coke has been deposited on the catalyst, followed by a second step in which the coke-loaded catalyst is contacted at a temperature of 350—700°C with an oxygen-containing gas until more than 50%w of the coke present on the catalyst has been removed.

The gallium present in the catalysts occurs exclusively in the crystalline silicate having the special structure and has been incorporated therein during the preparation of the silicate by crystallization from the aqueous mixture containing one or more gallium compounds.

The preparation of the crystalline gallium silicates used in the process according to the invention is carried out starting from an aqueous mixture containing the following compounds: one or more compounds of an alkali metal (M), one or more organic nitrogen compounds (RN) which contain an organic cation or from which an organic cation is formed during the preparation of the silicate, one or more silicon compounds, one or more gallium compounds and, if desired, one or more compounds of a trivalent metal Y. The preparation is suitably carried out by maintaining the mixture at an elevated temperature until the silicate has formed, and subsequently separating the silicate crystals from the mother liquor and washing, drying and calcining the crystals. In the aqueous mixture from which the silicates are prepared the various compounds should suitably be present in the following molar ratios expressed—with the exception of the organic nitrogen compounds—in moles of the oxides:

$M_2O:SiO_2=0.01$—0.35,
$RN:SiO_2=0.02$—1.0,
$SiO_2:Ga_2O_3=25$—400,
$Y_2O_3:Ga_2O_3<1$, and
$H_2O:SiO_2=5$—65.

In the preparation of the silicates the base mixture may very suitably be a mixture containing a quaternary ammonium compound as the organic nitrogen compound, a sodium compound as the alkali metal compound and amorphous silica as the silicon compound.

In the process according to the invention preference is given to the use of crystalline gallium silicates which have been prepared by crystallization from an aqueous mixture which, apart from possible impurities present in the reaction components, contains no compounds of a trivalent metal Y.

The silicates prepared as described hereinbefore contain alkali metal ions. By using suitable exchange methods these may be replaced by other cations, such as hydrogen ions or ammonium ions. The crystalline gallium silicates used in the process according to the invention preferably have an alkali metal content of less than 0.05%w. In the process according to the invention the crystalline gallium silicates may be used per se or in combination with a binder material, such as kaolin or bentonite.

Olefins having two, three or four carbon atoms per molecule can be converted at a relatively low temperature and in high yields into aromatic hydrocarbon mixtures by contacting the olefins with crystalline metal silicates having a similar structure as the gallium silicates used in the process according to the invention have.

A similar conversion into aromatic hydrocarbon mixtures of paraffins having two, three or four carbon atoms per molecule and of aliphatic hydrocarbon mixtures which consist more than 50%w of said paraffins

2

is much more difficult and requires considerably higher temperatures, which accounts for the important role played by cracking reactions and the low yields of $C_5^+$ hydrocarbons. In this conversion hydrogen is released. In view of the growing demand for hydrogen for a variety of purposes it is important that in the conversion as much as possible of the hydrogen becomes available as molecualr hydrogen and not in the form of hydrogen-rich by-products, such as methane.

It has been found that catalysts comprising crystalline gallium silicates with a $SiO_2/Ga_2O_3$ molar ratio between 25 and 100 have a very high activity as well as a very high $H_2$ and $C_5^+$ selectivity. Catalysts comprising crystalline gallium silicates with a $SiO_2/Ga_2O_3$ molar ratio between 100 and 250 have a relatively low activity and $C_5^+$ selectivity. The performance of all crystalline gallium silicates-comprising catalysts is greatly enhanced by subjecting them at least once to the above two-step treatment.

According as the catalysts are subjected to the two-step treatment more often, their performance in the conversion will improve. This improvement continues until a certain maximum level has been reached where further repetition of the two-step treatment ceases to produce any effect.

In the process according to the invention the starting material comprises paraffins having two, three or four carbon atoms per molecule or an aliphatic hydrocarbon mixture which consists more than 50%w of said paraffins. The paraffins with two, three or four carbon atoms per molecule which should constitute more than 50%w of the feed are ethane, propane, n-butane and isobutane. If the starting material is an aliphatic hydrocarbon mixture which, in addition to the paraffins mentioned, contains other aliphatic hydrocarbons as well, this mixture may contain, inter alia, methane, ethene, propene, butene, isobutene, butadiene and paraffins and olefins with five or more carbon atoms per molecule. In the process according to the invention the starting material preferred is a feed which consists more than 75%w, and in particular substantially completely, of one or more paraffins having three or four carbon atoms per molecule. A feedstock which is very suitable for use in the process is a mixture of paraffins with three and four carbon atoms per molecule obtained as a by-product in the production of mineral oil.

The liquid hydrocarbon mixtures obtained in the present conversion boil substantially in the gasoline range and have a very high octane number. They are therefore excellently suitable for use as motor gasoline or as mixing components for motor gasolines.

The process according to the invention is preferably carried out at a temperature of 350—700°C and in particular of 450—650°C, a pressure of 1—20 bar and in particular of 1—10 bar and a space velocity of 0.1—10 kg · kg$^{-1}$ · hour$^{-1}$ and in particular of 0.5—5 kg · kg$^{-1}$ · hour$^{-1}$.

In the process according to the invention the feed is contacted with a catalyst containing a crystalline gallium silicate which is defined, among other things, by the X-ray powder diffraction pattern which the silicate shows after one hour's calcination in air at 500°C. In this pattern the strongest lines should be the four lines mentioned in Table A. The complete X-ray powder diffraction pattern of a typical example of the present crystalline gallium silicates after one hour's calcination in air at 500°C is given in Table B.

TABLE B

| d(Å) int. | | Rel. int | d(Å) | Rel. |
|---|---|---|---|---|
| 11.1 | | 100 | 3.84 (D) | 57 |
| 10.0 | (D) | 70 | 3.72 (D) | 31 |
| 8.93 | | 1 | 3.63 | 16 |
| 7.99 | | 1 | 3.47 | <1 |
| 7.42 | | 2 | 3.43 | 5 |
| 6.68 | | 7 | 3.34 | 2 |
| 6.35 | | 11 | 3.30 | 5 |
| 5.97 | | 17 | 3.25 | 1 |
| 5.70 | | 7 | 3.05 | 8 |
| 5.56 | | 10 | 2.98 | 11 |
| 5.35 | | 2 | 2.96 | 3 |
| 4.98 (D) | | 6 | 2.86 | 2 |
| 4.60 | | 4 | 2.73 | 2 |
| 4.35 | | 5 | 2.60 | 2 |
| 4.25 | | 7 | 2.48 | 3 |
| 4.07 | | 2 | 2.40 | 2 |
| 4.00 | | 4 | | |

(D)=doublet.

Catalysts which are eligible for use in the process according to the invention are catalysts containing a crystalline gallium silicate having a $SiO_2/Ga_2O_3$ molar ratio of 25—250.

Since investigation has shown that application of the two-step treatment according to the invention to catalysts containing a crystalline gallium silicate having a $SiO_2/Ga_2O_3$ molar ratio lower than 60 produces catalysts whose performance in the present conversion is not superior to that obtained when applying said treatment to catalysts containing a crystalline gallium silicate having a $SiO_2/Ga_2O_3$ molar ratio of 60—100, it is preferred in the process according to the invention, in view of the fairly high cost of gallium, to use a catalyst containing a crystalline gallium silicate having a $SiO_2/Ga_2O_3$ molar ratio of at least 60.

As regards the number of times that the two-step treatment should be carried out in order to obtain a catalyst with acceptable or optimum performance in the present conversion, the following may be remarked.

In general the performance of catalysts containing a crystalline gallium silicate with a $SiO_2/Ga_2O_3$ molar ratio of at most 110 may be brought to an optimum level by subjecting the catalysts at most three times to the two-step treatment.

The minimum number of two-step treatments that is applied to catalysts containing a crystalline gallium silicate with a $SiO_2/Ga_2O_3$ molar ratio higher than 110 preferably is given by the formula

$$n = \frac{m - 100}{10},$$

on the understanding that if the afore-mentioned formula, in which n represents the minimum number of two-step treatments and m the $SiO_2/Ga_2O_3$ molar ratio of the silicate, produce a value for n which may be expressed as the sum of a natural number N and a fractional number smaller than 1, the preferred minimum number of times that the catalyst should be subjected to the two-step treatment is N+1.

The performance of latter catalysts which, by the application of the number of two-step treatments given by the formula, has been raised to a higher level, can be enhanced still further to attain an optimum level by increasing the number of two-step treatments. The investigation has revealed that the number of two-step treatments to which the catalyst preferably is subjected in order to attain optimum performance is about three times as much as the number of times (n, alternatively N+1) which, according to the formula, is the preferred minimum. For instance, in the case of catalysts containing crystalline gallium silicates with $SiO_2/Ga_2O_3$ molar ratios of 135, 165 or 195, acceptable performance is obtained by subjecting them to the two-step treatment four, seven or ten times, respectively, whilst in order to attain optimum performance, the treatment should be carried out about 12, 21 or 30 times, respectively. As can be seen from the above, both for achieving acceptable performance and for achieving optimum performance the two-step treatment should preferably be carried out more often according as the crystalline gallium silicate has a higher $SiO_2/Ga_2O_3$ molar ratio. In the process according to the invention both catalysts containing a crystalline gallium silicate with a high $SiO_2/Ga_2O_3$ molar ratio and catalysts in which the crystalline gallium silicate has a low $SiO_2/Ga_2O_3$ molar ratio may be used. The choice of $SiO_2/Ga_2O_3$ molar ratio is mainly determined by two factors, viz. the fairly high cost of gallium and the expense entailed in the two-step treatment. According as the crystalline gallium silicate present in the catalyst has a higher $SiO_2/Ga_2O_3$ molar ratio (viz. contains less gallium), the catalyst will be cheaper, but it will have to be subjected to the two-step treatment more often in order to carry its performance to an optimum level, which then leads to an increase of cost. If, when using the present catalysts on a technical scale, it is the object to limit the number of two-step treatments to which the catalyst has to be subjected in order to bring its performance to an optimum level, then this implies that in the process according to the invention preference is given to the use of catalysts containing a crystalline gallium silicate with a $SiO_2/Ga_2O_3$ molar ratio of at most 130.

It appears that the problem of catalysts containing a crystalline gallium silicate with a $SiO_2/Ga_2O_3$ molar ratio higher than 130 necessarily being preferred on account of their price, whereas on account of the expenses entailed in a large number of two-step treatments required to carry the performance of these catalysts to an optimum level, they are certainly not to be preferred, can be solved in an attractive way. The number of times that the two-step treamtent has to be carried out in order to carry their performance to a certain desired high level can be considerably decreased if, before being subjected to a succession of two-step treatments, the catalysts are exposed to calcination at a temperature of 600—1000°C for 2—8 hours. The preferred mininum number of times that catalysts which have been subjected to previous calcination of 600—1000°C have to be subjected to the two-step treatment in order to bring their performance in the present conversion to a preferred level depends, again, on the $SiO_2/Ga_2O_3$ molar ratio of the silicate present therein, and for catalysts in which the silicate has a $SiO_2/Ga_2O_3$ molar ratio higher than 130, the mininum number preferably is given by the formula

$$n = \frac{m-100}{30},$$

wherein n and m have the meanings mentioned hereinbefore. In this connection it should be noted that when the above formula produces a value for n which can be expressed as the sum of a natural number N and a fractional number smaller than 1, the minimum number of times that the catalyst should be subjected to the two-step treatment is preferably N+1.

Subjecting the pre-calcined catalyst to a number of two-step treatments which corresponds to n (alternatively N+1) of the formula leads to the production of a catalyst with acceptable performance in the present conversion. Just as in the case of the catalysts which have not been subjected to previous calcination at 600—1000°C, so in the case of the catalysts which have been subjected to such calcination, performance, having been brought to an acceptable level by the use of the number of two-step treatments indicated by the formula, can be further enhanced to attain an optimum level by an increase of the number of two-step treatments. The catalysts which have undergone previous calcination at 600—1000°C are preferably subjected to the two-step treatment in order to attain optimum performance about twice the number (n, alternatively N+1) which, according to the formula, is the preferred minimum required to achieve acceptable performance. For instance, in the case of the aforementioned catalysts, containing crystalline gallium silicates with $SiO_2/Ga_2O_3$ molar ratios of 135, 165 or 195, if they have been subjected to previous calcination at 600—1000°C, acceptable performance may be obtained by subjecting them to the two-step treatment twice, three or four times, respectively, whilst for attaining the optimum performance these catalysts should be subjected to the treatment about four, six or eight times, respectively. If, when using the present catalysts on a technical scale, it is the object—as in the case of the catalysts which have not undergone previous calcination at 600—1000°C—to limit the number of times that the catalyst is to be subjected to the two-step treatment in order to bring its performance to an optimum level to less than 10, then, in the process according to the invention when using catalysts which have undergone calcination at 600—1000°C preceding the succession of two-step treatments, the catalysts used by preference are those containing a crystalline gallium silicate with a $SiO_2/Ga_2O_3$ molar ratio of at most 220. When using catalysts which have undergone calcination at 600—800°C preceding the succession of two-step treatments, special preference is given to the use of catalysts containing a crystalline gallium silicate with a $SiO_2/Ga_2O_3$ molar ratio of 130—220.

In the first step of the two-step treatment basically any hydrocarbon or mixture of hydrocarbons may be used, provided that at a temperature of 350—700°C and in a period of less than 5 hours a coke deposition on the catalyst of 0.1—5%w can thereby be realized. For this purpose the hydrocarbon or the mixture of hydrocarbons used as the feed in the process according to the invention is very suitable.

The oxygen-containing gas used in the second step of the two-step treatment for the removal of more than 50%w of the coke present on the catalyst may very suitably be air. Preferably more than 75%w of the coke present on the catalyst and in particular substantially all the coke present on the catalyst is removed in the second step of the two-step treatment.

The invention is now elucidated with the aid of the following example.

Example

Four crystalline gallium silicates (silicates 1—4) were prepared by heating mixtures of NaOH, amorphous silica, $(C_3H_7)_4NOH$ and $Ga(NO_3)_3$ in water, in an autoclave under autogenous pressure, at 150°C for 24 hours. After cooling of the reaction mixtures the silicates formed were filtered off, washed with water until the pH of the wash water was about 8 and dried at 120°C. After one hour's calcination in air at 500°C silicates 1—4 had the following properties

a) an X-ray powder diffraction pattern substantially corresponding with that mentioned in Table B, and
b) a $SiO_2/Ga_2O_3$ molar ratio as mentioned in Table C.

TABLE C

| Silicate No. | $SiO_2/Ga_2O_3$ molar ratio |
|---|---|
| 1 | 70 |
| 2 | 120 |
| 3 | 180 |
| 4 | 350 |

The molar compositions of the aqueous mixtures from which silicates 1—4 were prepared may be rendered as follows:

$$x\ Na_2O \cdot 9\ (C_3H_7)_4NOH \cdot y\ Ga_2O_3 \cdot 25\ SiO_2 \cdot 450\ H_2O$$

wherein x and y have the values given in Table D.

TABLE D

| Silicate No. | x | y |
|---|---|---|
| 1 | 3 | 0.33 |
| 2 | 1 | 0.2 |
| 3 | 1 | 0.125 |
| 4 | 1 | 0.06 |

From silicates 1—4 were prepared silicates I—IV, respectively, by boiling silicates 1—4 with a 1.0 molar $NH_4NO_3$ solution, washing with water, boiling again with a 1.0 molar $NH_4NO_3$ solution and washing, drying at 120°C and calcination at 500°C.

Samples of silicates I—IV were repeatedly subjected to a two-step treatment comprising a step in which the silicate was contacted with n-butane for 30 minutes at a temperature of 600°C, a pressure of 1.5 bar and a space velocity of $8\ g \cdot g^{-1} \cdot h^{-1}$ and in which a quantity of coke ranging from 0.5 to 1.5%w was deposited on the silicate, followed by a second step in which the silicate was contacted with air for 1 hour at a temperature of 500°C and a pressure of 1.5 bar and in which 95—99%w of the coke present on the silicate was removed. From silicates I—IV were thus produced catalysts IA and IB, IIA—IID, IIIA—IIIE and IVA, respectively. In addition two samples of silicate III were first contacted with air for 1 hour at 700°C and then repeatedly subjected to the two-step treatment described hereinbefore. From silicate III were thus produced catalysts IIIF and IIIG.

Catalysts I—IB, II—IID, III—IIIG, IV and IVA were tested in eighteen experiments (Experiments 1—18) in the preparation of $C_5^+$ aromatic hydrocarbon mixtures starting from n-butane. The experiments were carried out in a reactor containing a fixed catalyst bed. All the experiments were carried out at a temperature of 550°C, a pressure of 1.5 bar and a space velocity of $2 \ kg \cdot kg^{-1} \cdot hour^{-1}$. The results of the experiments are listed in Table E. Table E also indicates how many times each silicate was subjected to the two-step treatment and the percentages of coke deposited on each silicate in the first steps of the two-step treatments.

TABLE E

| Experiment No. | 1 | 2 | 3 | 4 | 5 | 6 | 7 | 8 |
|---|---|---|---|---|---|---|---|---|
| Catalyst No. | I | IA | IB | II | IIA | IIB | IIC | IID |
| Number of times that silicate was subjected to two-step treatment | — | 3 | 5 | — | 3 | 5 | 9 | 15 |
| Percentage of coke deposited on silicate in first step of two-step treatment, %w | — | 1.0—1.5 | 1.0—1.5 | — | 0.8—1.3 | 0.8—1.4 | 0.8—1.5 | 0.8—1.5 |
| Conversion, %w | 90 | 96 | 97 | 56 | 81 | 89 | 94 | 95 |
| Product selectivity, %w on converted material | | | | | | | | |
| $H_2$ | 5.0 | 4.9 | 4.3 | 3.6 | 4.0 | 4.5 | 4.5 | 4.2 |
| $C_1-C_3$ | 45.3 | 35.0 | 35.4 | 65.0 | 45.4 | 37.5 | 29.7 | 30.2 |
| iso-$C_4°$ | 1.8 | 0.7 | 0.8 | 5.9 | 1.9 | 1.1 | 1.0 | 1.1 |
| $\Sigma C_4=$ | 1.0 | 0.3 | 3.0 | 4.9 | 1.3 | 0.8 | 0.9 | 0.8 |
| $C_5^+$ | 46.9 | 59.1 | 59.2 | 20.6 | 47.4 | 56.1 | 63.9 | 63.7 |

| Experiment No. | 9 | 10 | 11 | 12 | 13 | 14 | 15 | 16 | 17 | 18 |
|---|---|---|---|---|---|---|---|---|---|---|
| Catalyst No. | III | IIIA | IIIB | IIIC | IIID | IIIE | IIIF | IIIG | IV | IVA |
| Number of times that silicate was subjected to two-step treatment | — | 3 | 10 | 15 | 25 | 40 | 6* | 30* | — | 30 |
| Percentage of coke deposited on silicate in first step of two-step treatment, %w | — | 0.15—0.3 | 0.15—0.7 | 0.15—0.9 | 0.15—1.2 | 0.15—1.2 | 0.3—1.0 | 0.3—1.2 | — | 0.15—0.3 |
| Conversion, %w | 46 | 50 | 58 | 62 | 81 | 81 | 79 | 80 | 41 | 35 |
| Product selectivity, %w on converted material | | | | | | | | | | |
| $H_2$ | 1.8 | 1.9 | 2.3 | 3.9 | 5.3 | 4.9 | 5.4 | 5.3 | 2.0 | 1.9 |
| $C_1$—$C_3$ | 69.8 | 68.5 | 57.7 | 43.6 | 36.0 | 36.2 | 38.1 | 37.2 | 69.0 | 70.8 |
| iso-$C_4°$ | 5.6 | 6.0 | 5.9 | 5.8 | 2.5 | 2.7 | 3.2 | 2.8 | 4.0 | 3.0 |
| $\Sigma C_4=$ | 6.6 | 5.3 | 3.8 | 5.5 | 2.0 | 2.3 | 2.3 | 2.5 | 10.2 | 10.8 |
| $C_5^+$ | 16.2 | 18.3 | 30.3 | 41.2 | 54.2 | 53.9 | 51.0 | 52.2 | 14.8 | 13.5 |

*after previous treatment with air at 700°C.

# EP 0 107 876 B1

Of the experiments mentioned in Table E Experiments 2, 3, 5—8 and 10—16 are experiments according to the invention. These experiments were carried out using as the catalysts crystalline gallium silicates having a $SiO_2/Ga_2O_3$ molar ratio in the range between 25 and 250, which gallium silicates had been subjected to a number of two-step treatments according to the invention. These catalysts show a high activity and a high $H_2$ and $C_5^+$ selectivity. Comparison of the results of Experiments 7 and 8 (carried out using a crystalline gallium silicate having a $SiO_2/Ga_2O_3$ molar ratio of 120) shows that subjecting the gallium silicate nine times to the two-step treatment yields a catalyst of excellent performance, but that its performance is not markedly enhanced by repeating the two-step treatment 15 times. A similar conclusion may be drawn from the results of Experiments 13 and 14 (carried out using a crystalline gallium silicate having a $SiO_2/Ga_2O_3$ molar ratio of 180), which show that no marked improvement of the catalyst performance is to be seen when the two-step treatment is carried out 40 times instead of 25 times. Comparison of the results of Experiments 13 and 15 (carried out using a crystalline gallium silicate with a $SiO_2/Ga_2O_3$ molar ratio of 180) shows that the number of two-step treatments may be drastically reduced (from 25 to 6), if prior to the repeated two-step treatments the crystalline gallium silicate is subjected to calcination at 700°C. Comparison of the results of Experiments 15 and 16 (carried out using a crystalline gallium silicate with a $SiO_2/Ga_2O_3$ molar ratio of 180) shows that no marked enhancement of the catalyst performance is to be seen when, with the application of calcination at 700°C followed by a repeated two-step treatment, the number of two-step treatments is raised from 6 to 30.

Experiments 1, 4, 9, 17 and 18 fall outside the scope of the invention. They have been included in the patent application for comparison. Experiments 1, 4 and 9 were carried out using as catalyst a crystalline gallium silicate with a $SiO_2/Ga_2O_3$ molar ratio in the range between 25 and 250, which gallium silicates had not however been subjected to a two-step treatment according to the invention. In Experiment 10 (carried out using a crystalline gallium silicate with a $SiO_2/Ga_2O_3$ molar ratio of 180) the gallium silicate was subjected to the two-step treatment three times, whereas the formula which expresses the relation between preferred minimum number of times for the two-step treatment to be carried out and $SiO_2/Ga_2O_3$ molar ratio of the silicate stipulates that this number should be at least eight. By subjecting the catalyst only three times to the two-step treatment, one attains an improved performance of the catalyst, though comparison with Experiments 11—14 shows that the performance can be optimized by repeating the treatment several times. Experiments 17 and 18 were carried out using a crystalline gallium silicate with a $SiO_2/Ga_2O_3$ molar ratio higher than 250. As shown by the results of Experiments 17 and 18, the catalyst when not subjected to the two-step treatment shows an unacceptably poor performance, which will not be improved by subjecting the catalyst to the two-step treatment a large number of times.

## Claims

1. A process for the preparation of an aromatic hydrocarbon mixture, characterized in that paraffins with two, three or four carbon atoms per molecule or aliphatic hydrocarbon mixtures consisting more than 50%w of said paraffins are contacted with a catalyst comprising a crystalline gallium silicate which

a) has been prepared by crystallization starting from an aqueous mixture which contains one or more compounds of an alkali metal, one or more organic nitrogen compounds which contain an organic cation or from which an organic cation is formed during the preparation of the silicate, one or more silicon compounds, one or more gallium compounds and, if desired, one or more compounds of a trivalent metal Y chosen from the group formed by aluminium, iron, cobalt and chromium in such quantities that in the formula which represents the composition of the silicate expressed in moles of the oxides, the $SiO_2/Ga_2O_3$ molar ratio is 25—250 and the $Y_2O_3/Ga_2O_3$ molar ratio is lower than 1, and

b) after one hour's calcination in air at 500°C has an X-ray powder diffraction pattern in which the strongest lines are the four lines mentioned in Table A,

TABLE A

| d(Å) |
| --- |
| 11.1 ±0.2 |
| 10.0 ±0.2 |
| 3.84±0.07 |
| 3.72±0.06 |

which catalyst has been subjected once or several times to a two-step treatment involving a step in which the catalyst is contacted at a temperature of 350—700°C with a hydrocarbon or a mixture of hydrocarbons until in less than 5 hours 0.1—5%w of coke has been deposited on the catalyst, followed by a second step in which the coke-loaded catalyst is contacted at a temperature of 350—700°C with an oxygen-containing gas until more than 50%w of the coke present on the catalyst has been removed.

2. A process as claimed in claim 1, characterized in that the catalyst comprises a crystalline gallium silicate having a $SiO_2/Ga_2O_3$ molar ratio of at most 110 and that it is subjected to the two-step treatment at most three times.

10

3. A process as claimed in claim 1, characterized in that the catalyst comprises a crystalline gallium silicate with a $SiO_2/Ga_2O_3$ molar ratio higher than 110 and that the minimum number of two-step treatments to be carried out is given by the formula

$$n = \frac{m-100}{10},$$

on the understanding that if the aforementioned formula, in which n represents the minimum number of two-step treatments and m the $SiO_2/Ga_2O_3$ molar ratio of the silicate, produces a value for n which may be expressed as the sum of a natural number N and a fractional number smaller than 1, the minimum number of times that the catalyst should be subjected to the two-step treatment is N+1.

4. A process as claimed in claim 3, characterized in that the number of times that the catalyst is subjected to the two-step treatment is three times the number which the formula gives as the minimum.

5. A process as claimed in any one of claims 1—4, characterized in that the catalysts are exposed to calcination at a temperature of 600—1000°C for 2—8 hours before being subjected to a succession of two-step treatments.

6. A process as claimed in claim 5, characterized in that the catalyst comprises a crystalline gallium silicate with a $SiO_2/Ga_2O_3$ molar ratio higher than 130 which has been subjected to previous calcination at 600—1000°C, and that the minimum number of two-step treatments to be carried out is given by the formula

$$n = \frac{m-100}{30},$$

on the understanding that if the aforementioned formula, in which n represents the minimum number of two-step treatments and m the $SiO_2/Ga_2O_3$ molar ratio of the silicate, produces a value for n which may be expressed as the sum of a natural number N and a fractional number smaller than 1, the minimum number of times that the catalyst should be subjected to the two-step treatment is N+1.

7. A process as claimed in claim 6, characterized in that the catalyst comprises a crystalline gallium silicate having a $SiO_2/Ga_2O_3$ molar ratio higher than 130, but at most 220, and that the number of times that it is subjected to the two-step treatment following calcination at 600—1000°C is twice the number which the formula gives as the minimum.

8. A process as claimed in any one of claims 1—7, characterized in that it is applied to a feedstock more than 75%w of which consists of one or more paraffins having three or four carbon atoms per molecule.

9. A process as claimed in any one of claims 1—8, characterized in that in order to achieve the desired coke deposition in the first step of the two-step treatment, use is made of the feed.

10. A process as claimed in any one of claims 1—9, characterized in that it is carried out at a temperature of 350—700°C, a pressure of 1—20 bar and a space velocity of $0.1$—$10\, kg \cdot kg^{-1} \cdot h^{-1}$.

**Patentansprüche**

1. Ein Verfahren zur Herstellung einer aromatischen Kohlenwasserstoffmischung, dadurch gekennzeichnet, daß Paraffine mit zwei, drei oder vier Kohlenstoffatomen im Molekül oder aliphatische Kohlenwasserstoffmischungen, bestehend zu mehr als 50 Gewichtsprozent aus den genannten Paraffinen, mit einem Katalysator, enthaltend ein kristallines Galliumsilikat, kontaktiert werden, welches

a) durch Kristallisation, ausgehend von einer wäßrigen Mischung, welche eine oder mehrere Verbindungen eines Alkalimetalls, eine oder mehrere organische Stickstoffverbindungen, welche ein organisches Kation enthalten oder aus welchen ein organisches Kation während der Herstellung des Silikats gebildet wird, eine oder mehrere Siliciumverbindungen, eine oder mehrere Galliumverbindungen und gewünschtenfalls eine oder mehrere Verbindungen eines dreiwertigen Metalls Y, ausgewählt aus der Gruppe gebildet durch Aluminium, Eisen, Kobalt und Chrom, in solchen Mengen enthält, daß in der Formel, welche die Zusammensetzung des Silikats ausgedrückt in Mol der Oxide wiedergibt, das $SiO_2/Ga_2O_3$-Molverhältnis von 25:1 bis 250:1 beträgt und das $Y_2O_3/Ga_2O_3$-Molverhältnis weniger als 1 beträgt, hergestellt wurde, und

b) nach einstündigem Kalzinieren an Luft bei 500°C ein Röntgenpulver-Streudiagramm aufweist, in welchem die stärksten Linien die vier in der Tabelle A angegebenen Linien sind,

TABELLE A

| d($\mathring{A}$) |
| --- |
| 11,1 ±0,2 |
| 10,0 ±0,2 |
| 3,84±0,07 |
| 3,72±0,06 |

welcher Katalysator einmal oder mehrere Male einer zweistufigen Behandlung unterworfen wurde, welche einen Schritt umfaßt, bei welchem der Katalysator bei einer Temperatur von 350 bis 700°C mit einem Kohlenwasserstoff oder einer Mischung aus Kohlenwasserstoffen kontaktiert wird, bis in weniger als 5 Stunden 0,1 bis 5 Gewichtsprozent Koks auf dem Katalysator abgelagert sind, worauf ein zweiter Schritt folgt, in welchem der mit Koks beladene Katalysator bei einer Temperatur von 350 bis 700°C mit einem sauerstoffhaltigen Gas kontakiert wird, bis mehr als 50 Gewichtsprozent des auf dem Katalysator vorhandenen Koks entfernt sind.

2. Ein Verfahren wie in Anspruch 1 beansprucht, dadurch gekennzeichnet, daß der Katalysator ein kristallines Galliumsilikat mit einem Molverhältnis $SiO_2/Ga_2O_3$ von höchstens 110 umfaßt und daß er höchstens dreimal der Zweistufenbehandlung unterworfen worden ist.

3. Ein Verfahren wie in Anspruch 1 beansprucht, dadurch gekennzeichnet, daß der Katalysator ein kristallines Galliumsilikat mit einem molaren Verhältnis von $SiO_2/Ga_2O_3$ von mehr als 110 umfaßt und daß die Mindestzahl an Zweistufenbehandlungen die durchzuführen sind, durch die Formel

$$n = \frac{m - 100}{10}$$

wiedergegeben wird, mit der Maßgabe, daß, wenn die genannte Formel, in welcher n die Mindestzahl der Zweistufenbehandlungen und m das molare Verhältnis $SiO_2/Ga_2O_3$ des Silikats darstellt, einen Wert für n ergibt, der als Summe einer natürlichen Zahl N und einer Bruchzahl kleiner als 1 ausgedrückt werden kann, die Mindestzahl der Male, die der Katalysator der Zweistufenbehandlung unterworfen werden sollte, N+1 ist.

4. Ein Verfahren wie in Anspruch 3 beansprucht, dadurch gekennzeichnet, daß die Anzahl der Male, die der Katalysator der Zweistufenbehandlung unterworfen wird, dem dreifachen Wert der Zahl entspricht, die die Formel als Minimum angibt.

5. Ein Verfahren wie in einem der Ansprüche 1 bis 4 beansprucht, dadurch gekennzeichnet, daß die Katalysatoren einer Kalzinierung bei einer Temperatur von 600 bis 1000°C 2 bis 8 Stunden lang unterworfen werden, bevor sie einer Folge von Zweistufenbehandlungen unterworfen werden.

6. Ein Verfahren wie in Anspruch 5 beansprucht, dadurch gekennzeichnet, daß der Katalysator ein kristallines Galliumsilikat mit einem Molverhältnis $SiO_2/Ga_2O_3$ von mehr als 130 umfaßt, welches einer vorhergehenden Kalzinierung bei 600 bis 1000°C unterworfen wurde, und daß die Mindestzahl der durchzuführenden Zweistufenbehandlungen durch die Formel

$$n = \frac{m - 100}{30}$$

wiedergegeben wird, mit der Maßgabe, daß, wenn die genannte Formel, in welcher n die Mindestzahl der Zweistufenbehandlungen darstellt und m das molare Verhältnis $SiO_2/Ga_2O_3$ des Silikats darstellt, einen Wert für n ergibt, der als Summe aus einer natürlichen Zahl N und einer Bruchzahl kleiner als 1 ausgedrückt werden kann, die die Mindestzahl der Male, die der Katalysator einer Zweistufenbehandlung unterworfen werden sollte, N+1 ist.

7. Ein Verfahren wie in Anspruch 6 beansprucht, dadurch gekennzeichnet, daß der Katalystor ein kristallines Galliumsilikat mit einem Molverhältnis $SiO_2/Ga_2O_3$ von mehr als 130, höchstens aber 220 umfaßt, und daß die Anzahl der Male, die er einer Zweistufenbehandlung gefolgt von einer Kalzinierung bei 600 bis 1000°C unterworfen wird, dem zweifachen Wert der Zahl entspricht, die die Formel als Minimum angibt.

8. Ein Verfahren, wie in einem der Ansprüche 1 bis 7 beansprucht, dadurch gekennzeichnet, daß es auf ein Einsatzmaterial angewendet wird, das zu mehr als 75 Gewichtsprozent aus einem oder mehreren Paraffinen mit drei oder vier Kohlenstoffatomen pro Molekül besteht.

9. Ein Verfahren wie in einem der Ansprüche 1 bis 8 beansprucht, dadurch gekennzeichnet, daß zwecks Erreichen der gewünschten Koksablagerung in der ersten Stufe der Zweistufenbehandlung von dem Einsatzmaterial Gebrauch gemacht wird.

10. Ein Verfahren wie in einem der Ansprüche 1 bis 9 beansprucht, dadurch gekennzeichnet, daß es bei einer Temperatur von 350 bis 700°C, einem Druck von 1 bis 20 bar und einer Raumgeschwindigkeit von 0,1 bis 10 kg · kg$^{-1}$ · h$^{-1}$ durchgeführt wird.

**Revendications**

1. Un procédé pour la préparation d'un mélange d'hydrocarbures aromatiques, caractérisé en ce que des paraffines présentant deux, trois ou quatre atomes de carbone par molécule ou des mélanges d'hydrocarbures aliphatiques constitués de plus de 50% en poids desdites paraffines sont mises en contact avec un catalyseur comportant un silicate crystallin de gallium qui

a) a été préparé par cristallisation en partant d'une solution aqueuse qui contient un ou plus d'un

# EP 0 107 876 B1

composé de métal alcalin, ou un plus d'un composé organique azoté qui contient un cation organique ou à partir duquel un cation organique est formé pendant la préparation du silicate, un ou plus d'un composé du silicium, un ou plus d'un composé d'un métal trivalent Y chois dans le groupe constitué par l'aluminium, le fer, le cobalt et le chrome en des quantités telles que, dans la formule qui représente la composition du silicate exprimés en moles d'oxydes, le rapport molaire $SiO_2/Ga_2O_3$ est de 25—250 et le rapport molaire $Y_2O_3/Ga_2O_3$ est inférieur à 1, et

b) après une calcination d'une heure à l'air à 500°C présente un diagramme de diffraction aux rayons X sur poudre dans lequel les raies les plus fortes sont les quatre raies mentionnées sur le tableau A,

TABLEAU A

$d(\text{Å})$

11,1 ±0,2
10,0 ±0,2
3,84±0,07
3,72±0,06

lequel catalyseur a été soumis une ou plusieurs fois à un traitement en deux étapes, comportant une étape dans laquelle le catalyseur est mis en contact à une température de 350—700°C avec un hydrocarbure ou un mélange d'hydrocarbures jusqu'à ce que en moins de 5 heures, 0,1—5% de coke ait été déposé sur le catalyseur, suivi par une seconde étape dans laquelle le catalyseur chargé sur coke est mis en contact à une température de 350—700°C avec un gaz renfermant de l'oxygène jusqu'à ce que plus de 50% en poids du coke présent sur le catalyseur ait été éliminé.

2. Un procédé tel que revendiqué dans la revendication 1, caractérisé en ce que le catalyseur comprend du silicate cristallin de gallium présentant un rapport molaire $SiO_2/Ga_2O_3$ d'au plus 110 et qu'il est soumis au plus trois fois au traitement en deux étapes.

3. Un procédé tel que revendiqué dans la revendication 1, caractérisé en ce que le catalyseur comprend un silicate cristallin de gallium avec un rapport molaire $SiO_2/Ga_2O_3$ supérieur à 110 et que le nombre minimum de traitement en deux étapes à mettre en oeuvre est donné par la formule

$$n = \frac{m - 100}{10},$$

sous la condition que, si la formule mentionnée ci-dessus, dans laquelle n représente le nombre minimal de traitement en deux étapes et m le rapport molaire $SiO_2/Ga_2O_3$ du silicate, donne une valeur pour n qui peut être exprimée comme la somme d'un nombre naturel N et d'un nombre fractionnaire plus petit que 1, le nombre minimum de fois que le catalyseur doit être soumis au traitement en deux étapes est N+1.

4. Un procédé tel que revendiqué dans la revendication 3, caractérisé en ce que le nombre de fois que le catalyseur est soumis au traitement en deux étapes est trois fois le nombre que la formule donne comme minimum.

5. Un procédé tel que revendiqué dans l'une quelconque des revendications 1 à 4, caractérisé en ce que les catalyseurs sont exposés à une calcination à une température de 600 à 1000°C pendant 2 à 8 heures avant d'être soumis à une succession de traitements en deux étapes.

6. Un procédé tel que revendiqué dans la revendication 5, caractérisé en ce que le catalyseur comprend un silicate cristallin de gallium avec un rapport molaire supérieur à 130, qui a été soumis à une calcination préalable entre 600 et 1000°C, et que le nombre minimum de traitement en deux étapes à mettre en oeuvre est donné par la formule

$$n = \frac{m - 100}{30},$$

sous la condition que, si la formule mentionnée ci-dessus, dans laquelle n représente le nombre minimum de traitement en deux étapes et m le rapport molaire $SiO_2/Ga_2O_3$ du silicate, donne une valeur pour n qui peut être exprimée comme la somme d'un nombre naturel N et d'un nombre fractionnaire plus petit que 1, le nombre minimum de fois que le catalyseur doit être soumis au traitement à deux étapes est N+1.

7. Un procédé tel que revendiqué dans la revendication 6, caractérisé en ce que le catalyseur comprend un silicate cristallin de gallium présentant un rapport molaire $SiO_2/Ga_2O_3$ supérieur à 130, mais d'au plus 220, et que le nombre de fois qu'il est soumis au traitement à deux étapes suivant la calcination entre 600 et 1000°C est deux fois le nombre que la formule donne comme minimum.

8. Un procédé tel que revendiqué dans l'une quelconque des revendications 1 à 7, caractérisé en ce qu'il est appliqué à une charge dont plus de 75% en poids est constitué d'une ou de plus d'une paraffine présentant trois ou quatre atomes de carbone par molécule.

13

9. Un procédé tel que revendiqué dans l'une quelconque des revendications 1 à 8, caractérisé en ce que afin d'obtenir le dépôt de coke désiré dans la première étape du traitement en deux étapes, on utilise la charge.

10. Un procédé tel que revendiqué dans l'une quelconque des revendications 1 à 9, caractérisé en ce qu'il est réalisé à une température de 350—700°C, une pression de 1—20 bars et une vitesse spatiale de $0,1—10 \, kg \cdot kg^{-1} \cdot h^{-1}$.